# EUROPEAN PATENT APPLICATION

(11) **EP 1 199 077 A1**
(43) Date of publication of application: **24.04.2002**
(21) Application number: 00309207.9
(22) Date of filing: 19.10.2000
(51) Int. Cl.: A61K 31/424, A61K 9/19

(54) **Stable compositions of oxapenem-3-carboxylic acids by Co-lyophilisation with pharmaceutical carriers**

(71) Applicant: Amura Limited, Cambridge CB4 OW5 (GB)
(72) Inventor: Pfaendler, Hans Rudolf, 82131 stockdorf (DE)
(74) Representative: Geering, Keith Edwin

(57) **Abstract**

According to the present invention there is provided a pharmaceutical composition comprising a co-lyophilizate of: a pharmaceutical carrier; and an active ingredient of formulae I and II or a pharmaceutically acceptable salt thereof, 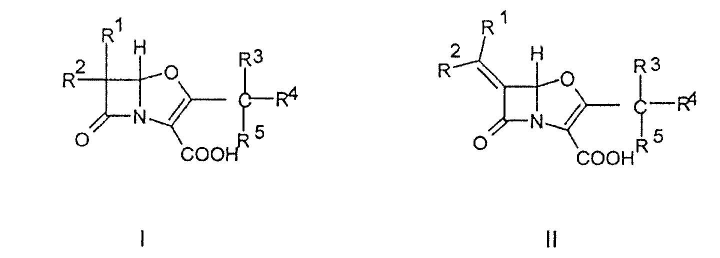 wherein R¹ and R², independently of one another, denote hydrogen, or pharmaceutically acceptable groups which have 1 to 10 carbon atoms and are bonded to the remaining part of the molecule via carbon-carbon single bonds and in which R³, R⁴ and R⁵, independently of one another, denote pharmaceutically acceptable groups which are bonded to the exocyclic, allylic carbon atom via carbon atoms.

## Description

This invention relates to stable pharmaceutical compositions. In particular, it relates to antibacterially active oxapenem-3-carboxylic acids of the general formula I and II and their pharmaceutically acceptable salts. 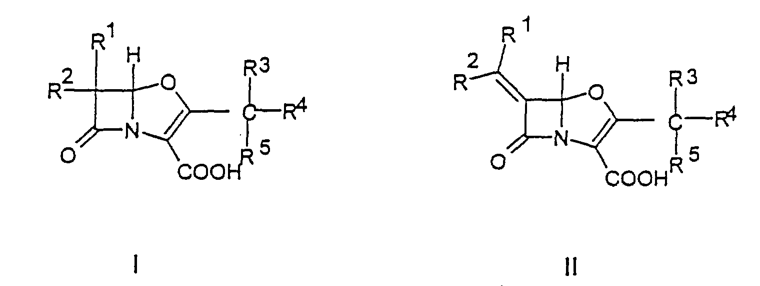 wherein R¹ and R², independently of one another, denote hydrogen, or pharmaceutically acceptable groups which have 1 to 10 carbon atoms and are bonded to the remaining part of the molecule via carbon-carbon single bonds and in which R³, R⁴ and R⁵, independently of one another, denote pharmaceutically acceptable groups which are bonded to the exocyclic, allylic carbon atom via carbon atoms. The stable pharmaceutical compositions are co-lyophilizates with pharmaceutically acceptable carriers.

The invention furthermore relates to the process for the preparation of such co-lyophilizates and methods of treatment in which these co-lyophilizates are administered if, for example, an antibiotic or a β-lactamase inhibition is indicated.

In one aspect, the invention relates to stable pharmaceutical compositions of 6-unsubstituted, 6-monosubstituted or 6,6-disubstituted 1-oxapenem-3-carboxylic acids and there pharmaceutically acceptable salts of the general structural formulae I and II 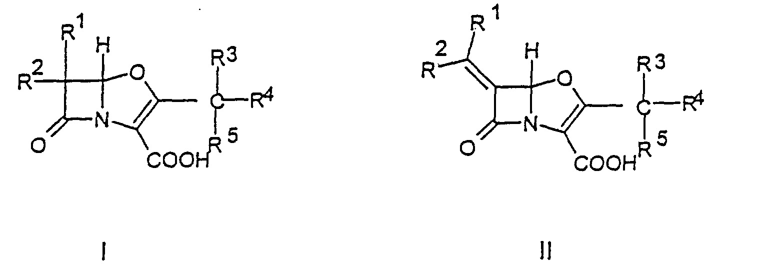 In which R¹ and R² independently of one another, are selected from: hydrogen, or the pharmaceutically acceptable groups which are bonded to the remaining part of the molecule by C-C single bonds and which contain: substituted or unsubstituted alkyl, alkenyl, alkinyl, cycloalkyl, alkylcycloalkyl, alkylcycloalkenyl, cycloalkylalkyl, alkenylcycloalkyl, cycloalkenylalkyl, aryl, aralkyl, aralkenyl, aralkinyl, carboxyl or cyano, where the foregoing alkyl, alkenyl or alkinyl molecular parts contain 1 to 6 carbon atoms, and the cycloalkyl or cycloalkenyl molecule parts contain 3 to 6 carbon atoms and the aryl molecular parts contain 6 to 10 carbon atoms, heteroaryl, heteroaralkyl, heteroaralkenyl, heteroaralkinyl, alkylheteroaryl, heterocyclyl, heterocyclylalkyl, heterocyclylalkenyl, heterocyclylalkinyl, or alkylheterocyclyl, where the foregoing alkyl, alkenyl or alkinyl molecular parts contain 1 to 6 carbon atoms and the heteroaromatic or heterocyclic molecule part is monocyclic or bicyclic and contains 3 to 10 ring atoms, of which one or more are selected from the series comprising: oxygen, sulfur and nitrogen, and where the substituents of the abovementioned groups may be: protected or unprotected hydroxyl, hydroxyalkoxy, aminoalkoxy, amidinoalkoxy, alkoxy, acyloxy, aryloxy, heteroaryloxy, heterocyclyloxy, carbamoyl, carbamoyloxy, thiocarbamoyl, thiocarbamoyloxy, alkylcarbamoyloxy, alkylthiocarbamoyloxy, mercapto, alkylthio, hydroxyalkylthio, aminoalkylthio, amidinoalkylthio, acylthio, arylthio, alkylheteroarylthio, hydroxyalkylheteroarylthio, heterocyclylthio, carbamoylthio, alkylcarbamoylthio, thiocarbamoylthio or alkylthiocarbamoylthio, protected or unprotected amino or monoalkylamino, dialkylamino, oxo, protected or unprotected oximino or alkylamino, tetraalkylammonium, cycloalkylamino, arylamino, heteroarylamino, heterocyclylamino, acylamino, amidino, alkylamidino, guanidino, alkylguanidino, carbamoylamino, alkylcarbamoylamino, thiocarbamoylamino, alkylthiocarbamoylamino, nitro, chloro, bromo, fluoro, iodo, azido, cyano, alkylsulphinyl, alkylsulphonyl, sulphonamido, sulphamoyloxy, alkylsulfonyloxy, or protected or unprotected sulpho, sulphoxy or carboxyl, where the substituents, independently of one another, occur once or several times and their alkyl molecule part contains 1 to 6 carbon atoms and their aryl molecule part contains 6 to 10 carbon atoms, and where the heteroaromatic or heterocyclic molecule part is monocyclic or bicyclic and contains 3 to 10 ring atoms, or which one or more are selected from the series comprising: oxygen, sulphur and nitrogen, and characterized in that R³, R⁴ and R⁵, independently of one another, are selected from the abovementioned, pharmaceutically acceptable groups which are bonded to the remaining part of the molecule via carbon-carbon single bonds and containing a pharmaceutical carrier, characterized in that the active ingredient and the carrier form a co-lyophilizate.

The protecting groups for the abovementioned protected substituents are known in the art and are described, e.g in T.W. Greene, "Protective Groups in Organic Synthesis", Wiley, N.Y., 1981.

Oxapenem-3-carboxylic acids and their pharmaceutically acceptable salts constitute a potent class of nonclassical antibiotics and β-lactamase inhibitors. It was described (EP 0301394, corresponding to US Pat. 5,096,899 and EP 0362622 corresponding to US Pat. 5,108,747) that increased stability towards hydrolysis was achieved by bulky 2-substituents. E.g. a 2-tert-butyl substituent increased the half-life of hydrolysis more than 30-fold as compared to the 2-methyl substituent. The higher stability in aqueous solutions was an important prerequisite for the use of oxapenem-3-carboxylic acids and their pharmaceutically acceptable salts in the removal of bacteria in human and veterinary therapy and in inanimate systems.

A preferred class of compounds within the oxapenem-3-carboxylic acids are those carrying a 6-hydroxyethyl group. Such compounds are described e.g. in Bioorg. Med. Chem. Lett. 3, (11), 2211 (1993). They have high potency as antibiotics and β-lactamase inhibitors.

Despite the increased stability of oxapenem-3-carboxylic acids and their pharmaceutically acceptable salts in aqueous solutions, a major obstacle arose by the low shelf-life of oxapenem-3-carboxylic acids and their pharmaceutically acceptable salts in their neat state. Although they can be stored over years at very low temperature (-80 °C) without any observable decomposition, it was found that storage at room temperature led to extensive decomposition within a few weeks. This short shelf-life was a serious obstacle for the use of oxapenem-3-carboxylic acids and their pharmaceutically acceptable salts in practical treatment of infectious diseases. Consequently, the improvement of shelf stability was essential for their applicability in human and veterinary therapy and in inanimate systems.

Residual water constitues a major factor for the degradation of pharmaceuticals. With many pharmaceuticals or vitamins this problem is solved by careful dehydrating and/or using special containers, e.g. fitted with a drying cap containing silica gel or the like. These techniques are known in the art.

With the oxapenem-3-carboxylic acids and their pharmaceutically acceptable salts careful drying and the use of drying agents or a drying cap during storage indeed led to a higher shelf stability when compared to that of the non-dried lyophilized compounds, but this procedure did not solve the imposed storage problem entirely. In fact, the carefully dried samples, still had insufficient shelf stability and were partly decomposed upon storage at room temperature within a few weeks, even when drying agents or a dry cap were used.

Antibacterial compositions comprising an antibacterially effective amount of an oxapenem-3-carboxylic acid or its pharmaceutically acceptable salts and a pharmaceutical excipient were known by prior art and have also been described in the above-mentioned patents. These compositions were prepared by simply mixing the active component with the pharmaceutical excipient lactose or maize starch.

Thus, a pure oxapenem-carboxylic acid was thoroughly mixed in a mortar with the pharmaceutical excipient lactose and the obtained mixture was carefully dried and stored at room temperature for six weeks. However, there was no improvement on shelf stability of such pharmaceutical compositions (prepared by mixing) when compared to that of the neat oxapenem-3-carboxylic acids or that of their pharmaceutically acceptable salts, revealing that an ideal homogeneous distribution of the antibiotic in the carrier cannot be achieved using the mixing procedure known by prior art.

However and surprisingly, it was found that the shelf stability of oxapenem-3-carboxylic acids is substantially improved, when the oxapenem-3-carboxylic acid or their pharmaceutically acceptable salts were co-loyphilized with a pharmaceutically acceptable carrier. Such pharmaceutical compositions were prepared by lyophilisation of an aqueous solution containing the oxapenem-3-carboxylic acids or their pharmaceutically acceptable salts and a pharmaceutically acceptable carrier. The obtained co-lyophilizate, after drying and storage over drying agents or using a drying cap, remained completely stable and retained its full biological activity after a six weeks storage at room temperature.

The present invention provides a pharmaceutical composition of oxapenem-3-carboxylic acids and their pharmaceutically acceptable salts having a high shelf stability which is important for their use in human and veterinary therapy and in inaminate systems. Such stable pharmaceutical compositions of oxapenem-3-carboxylic acids and their pharmaceutically active salts have not been described in prior art.

According to the present invention there is provided a pharmaceutical composition comprising a co-lyophilizate of: a pharmaceutical carrier; and an active ingredient of formulae I and II or a pharmaceutically acceptable salt thereof, 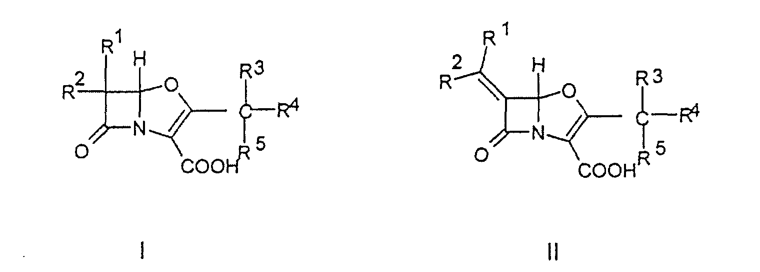 wherein R¹ and R², independently of one another, denote hydrogen, or pharmaceutically acceptable groups which have 1 to 10 carbon atoms and are bonded to the remaining part of the molecule via carbon-carbon single bonds and in which R³, R⁴ and R⁵, independently of one another, denote pharmaceutically acceptable groups which are bonded to the exocyclic, allylic carbon atom via carbon atoms.

According to the present invention in a further aspect there is provided a method of improving the shelf life of a pharmaceutical composition which includes an active ingredient comprising the step of lyophilizing a pharmaceutical carrier and the active ingredient. Co-lyophilization of oxapenem-3-carboxylic acids with a pharmaceutical carrier offers several advantages. First, it allows an ideal and homogeneous distribution of the active component in the carrier, avoiding undesired intermolecular degradation reactions during storage of the oxapenem-3-carboxylic acids or their pharmaceutically acceptable salts, second, it reduces the possible degradation of the (hygroscopic) active component during the formulation procedure and third, the obtained voluminous powder is dissolved more readily and completely when dissolved in cold water, e.g. in the preparation of solutions for parenteral application.

Co-lyophilisation of the oxapenem-carboxylic acids and their pharmaceutically acceptable salts can be carried out with one or several pharmaceutically acceptable water soluble carriers such as lactose, saccharose, glucose and the like.

Carriers which are not or only slightly water soluble, e.g. com starch or maize starch can be used. The type of carrier is mainly determined by the type of application for therapeutic use. For parenteral application water-soluble carriers are preferred. The carriers are solid and non-toxic, preferably digestible substances that are unreactive towards the active ingredient.

Additional ingredients and/or additional active constituents may also be added for co-lyophilisation. Examples for such ingredients are magnesium stearate, dicalcium phosphate or amino acids, e.g. lysine and the like.

Examples of additional active constituents are antibiotics, for example β-lactam antibiotics such as ceftazidime, cefotaxime, ceftriaxone, cefixime, cefaclo, cefuroxime, amoxycillin, piperacillin and the like.

The co-lyophilized carrier and oxapenem-3-carboxylic acids or their pharmaceutically acceptable salts can also be mixed (after co-lyophilization) with additional ingredients or active constituents.

Pharmaceutically acceptable salts of oxapenem-3-carboxylic acids are known in the art and are described in EP 0301394, corresponding to US Pat. 5,096,899.

In the preparation of oxapenem-3-carboxylic acids and their pharmaceutically acceptable salts the products were prepared by removing the ester protection groups in the final reaction step. A preferred way of preparation was the hydrogenolysis of p-nitrobenzyl esters of oxapenem-3-carboxylic acids in organic solvents such as ethyl acetate or in mixtures of organic solvents such as ethyl acetate and water. The oxapenem-3-carboxylic acids or their pharmaceutically acceptable salts are then extracted into the water phase. A preferred method for the stable preparations according to the invention is to dissolve the solid carrier in this water phase or to add an aqueous solution of the carrier (Method A). An alternative method of preparation of the stable pharmaceutical composition according to the invention consists of carrying out the deprotection in the presence of an aqueous solution containing the above-mentioned carrier already. Thus, a solution containing the oxapenem-3-carboxylic acid or their pharmaceutically acceptable salts and the inert carrier is obtained, ready for co-lyohilization.

An other preferred alternative is to prepare and co-lyophilize an aqueous solution containing the neat oxapenem-3-carboxylic acids or their pharmaceutically acceptable salts and the pharmaceutically acceptable carrier (Method B).

The amounts of pharmaceutically acceptable carrier relative to that of oxapenem-3-carboxylic acid or the pharmaceutically accceptable salt thereof is not very critical and depends on the type of application for therapeutic use. For oral application a 1: 1 up to a 20 : 1 ratio of carrier to oxapenem-3-carboxylic acid or the salt thereof is possible. The preferred range is from 2 : 1 up to 10 : 1. For parenteral application a ratio of 1 : 1 up to 8 : 1 is possible, the preferred range is from 2 : 1 up to 5 : 1.

For oral application the co-lyophilizate can further be mixed with effervescent ingredients such as sodium hydrogen carbonate and tartaric or citric acid and the like for the preparation of effervescent lemonades or elixirs.

The stable pharmaceutical preparations are prepared as follows:

### Example 1 (Method A)

Stable pharmaceutical preparation of (5R,6R,1'R)-3-(4-amino-1,1-dimethylbutyl)-6-(1'-hydroxyethyl)-7-oxa-1-azabicyclo[3.2.0]hept-2-ene-2-carboxylic acid in lactose

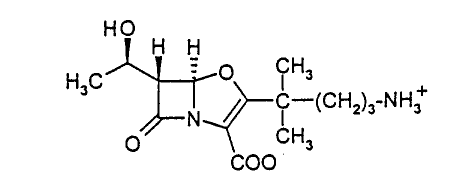

A solution of 3.79 g (8.25 mmol) p-nitrobenzyl (5R,6R,1'R) 3-(4-azido-1,1-dimethylbutyl)-6-(1 '-hydroxyethyl)-1-azabicyclo[3.2.0]hept-2-ene-2-carboxylate in 30 ml ethyl acetate was added at 0 °C via a syringe through a rubber septum to a prehydrogenated mixture of 3.1 g palladium on charcoal (10 %) in 120 ml of ethyl acetate and 60 ml of water. After a reaction time of 70 minutes at 0 °C, 840 ml of hydrogen have been taken up (theoretical amount 740 ml). The reaction mixture was filtered through a G5 glass filter of 10 cm diameter, the residue washed with 30 ml of cold water and 30 mol of cold ethyl acetate and the ethyl acetate layer removed from the combined filtrates. The aqueous layer was washed at 0 °C with 50 ml of cold ethyl acetate and 50 ml of cold toluene and the resulting aqueous colloidal solution pressed through a membrane filter using a syringe where upon the layers separated. The aqueous layer was evacuated in high vacuum in order to remove residual organic solvents. To the aqueous solution (89.8 ml) a cold solution containing 7.20 g lactose monohydrate in 180 ml water was added and 3ml portions of the resulting solution filled into glass ampoules. The content was frozen in a dry ice-acetone bath and the water removed in a lyophilizer at -25 °C during 4 days at 0.01 mbar. The resulting white powder was dried in a dessicator over phosphorous pentoxide overnight at 0.001 mbar and room temperature leaving 98.3 mg of a white powder in each ampoule. UV spectroscopy in water at 262 nm revealed a content of 18.2 mg of title compound and 80.1 mg of lactose in each ampoule. The ampoules were filled with dry nitrogen and sealed or alternatively stored over drying agents.

### Example 2

### Neat (5R,6R,1'R)-3-(4-amino-1,1-dimethylbutyl)-6-(1'-hydroxyethyl)-7-oxa-1-azabicyclo[3.2.0]hept-2-ene-2-carboxylic acid

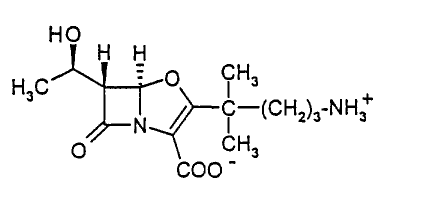

Following the procedure given in Example 1 from 4.0 g (8.71 mmol) p-nitrobenzyl (5R,6R,1'R) 3-(4-azido-1,1-dimethylbutyl)-6-(1'-hydroxyethyl)-1-azabicyclo[3.2.0]hept-2-ene-2-carboxylate a total of 1.99 g (77 %) of neat title compound as a white powder was obtained after simple lyophilization at -25 °C (without lactose) and after overnight drying at 0.001 bar and room temperature over phosphorous pentoxide. The ampoules were filled with dry nitrogen, sealed and kept at -80 °C.

### Example 3 (Method B)

### Stable pharmaceutical preparation of (5R,6R,1'R)-3-(4-amino-1,1-dimethylbutyl)-6-(1'-hydroxyethyl)-7-oxa-1-azabicyclo[3.2.0]hept-2-ene-2-carboxylic acid in lactose

10.7 mg of neat title compound and 40.0 mg of lactose monohydrate were dissolved in 1.5 ml of cold distilled water in a glass ampoule and immediately frozen in a dry ice acetone bath and the solution lyophilized at -25 °C in high vacuum (0.01 mbar) for 3 days. The white powder was dried overnight in high vacuum (0.001 mbar) over phosphorous pentoxide at room temperature. The white voluminous co-lyophilizate weighed 49.6 mg. UV spectroscopy at 262 nm in water indicated a content of the title compound of 9.6 mg and 40 mg of lactose.

### Example 4

### Shelf stability determinations

Several samples in glass ampoules were kept without and over drying agents for 42 days in the dark at room temperature. The content of (5R, 6R,1'R)-3-(4-amino-1,1-dimethylbutyl)-6-(1'-hydroxyethyl)-7-oxa-1-azabicyclo[3.2.0]hept-2-ene-2-carboxylic acid was determined by UV spectroscopy in water at λₘₐₓ = 262 nm (ε = 5000) and by plate bioassay. The appearance of the lyophilized powder and the solubility behaviour in water were also recorded.

The plate bioassays were carried out in sterile polystyrene dishes of 8.5 cm diameter with 10 ml of sterile Difco Nutrient Agar using Escherichia coli (inocculum ca. 10 000 cells). The inhibition diameters were compared with those obtained with 30, 25, 20, 15, 10 and 5 µg of pure title compound on a separate dish and the amount of active material calculated.

| Storage condition 42 days | drying agent | content by UV method | content by bioassay | appearance | solubility in H₂O |
|---|---|---|---|---|---|
| neat, -80 °C | none | 100 | 100 | white | +++ |
| neat, R.T | none | 67 | 40 | yellow | + |
| neat, R.T., vacuum | P₂O₅ | 76 | 83 | yellow | ++ |
| neat, R.T. | SiO₂ | 82 | 66 | yellow | ++ |
| mixed with lactose (1 : 4), R.T. | none | 74 | 45 | pale yellow | ++ |
| mixed with lactose, (1 : 4), R.T. | SiO₂ | 75 | 59 | pale yellow | + |
| co-lyophilized with lactose (1 : 4), R.T. | none | 92 | 96 | white | +++ |
| co-lyophilized with lactose, (1 : 4), R.T., vacuum | P₂O₅ | 100 | 100 | white | +++ |
| co-lyophilized with lactose (1 : 4), R.T. | SiO₂ | 100 | 100 | white | +++ |

### Example 5

### Production of pharmaceutical preparations

A unit dose form is prepared by mixing 300 mg of the (4 : 1) co-lyophilizate of lactose monohydrate and (5R,6R,1'R)-3-(4-amino-1,1-dimethylbutyl)-6-(1'-hydroxyethyl)-7-oxa-1-azabicyclo[3.2.0]hept-2-ene-2-carboxylic acid with 120 mg of cefaclor and 5 mg of magnesium stearate and the 425 mg mixture are added to a gelatine No. 3 capsule. Similarly, if co-lyophilizate of a higher content of oxapenem-3-carboxylic acid is used, other dose forms may be prepared likewise and filled into No. 3. gelatin capsules; and should it be necessary to mix more than 425 mg of costituents together, larger capsules, and also compressed tablets and pills, may also be produced. The following examples illustrate the production of pharmaceutical preparations

**Table**

| | |
|---|---|
| (4 : 1) co-lyophilizate of lactose monohydrate and (5R,6R,1'R)-3-(4-amino-1,1-dimethylbutyl)-6-(1'-hydroxyethyl)-7-oxa-1-azabicyclo[3.2.0]hept-2-ene-2-carboxylic acid | 625 mg |
| Cefaclor | 250 mg |
| Maize starch V.S.P. | 200 mg |
| Dicalcium phosphate | 60 mg |
| Magnesium stearate | 60 mg |

The co-lyophilizate and the other active constituent are mixed with the dicalcium phosphate and about half of the maize starch. The mixture is then granulated and coarsely sieved. It is dried at 45 °C and resieved through sieves of mesh width 1.0 mm (No. 16 screens). The remainder of the maize starch and the magnesium stearate are added and the mixture is compressed to form tablets each weighing 1195 mg and having a diameter of 1.27 cm (0.5 in.).

| Parenteral solution | |
|---|---|
| Ampoule | |
| (4 : 1) co-lyophilizate of lactose monohydrate and (5R,6R,1'R)-3-(4-amino-1,1-dimethylbutyl)-6-(1'-hydroxyethyl)-7-oxa-1-azabicyclo[3.2.0]hept-2-ene-2-carboxylic acid | 1250 mg |
| | |
| Ceftazidime | 500 mg |
| | |
| Sterile water (is added from a separate ampoule using a syringe immediately before use) | 5 ml |

| Ophtalmic solution | |
|---|---|
| (4 : 1) co-lyophilizate of lactose monohydrate and (5R,6R,1'R)-3-(4-amino-1,1-dimethylbutyl)-6-(1'-hydroxyethyl)-7-oxa-1-azabicyclo[3.2.0]hept-2-ene-2-carboxylic acid | 625 mg |
| | |
| Ceftazidime | 250 mg |
| | |
| Hydroxypropylmethylcellulose | 15 mg |
| | |
| Sterile water (is added from a separate ampoule using a syringe immediately before use | 2 ml |

| Otic solution | |
|---|---|
| (4 : 1) co-lyophilizate of lactose monohydrate and (5R,6R,1'R)-3-(4-amino-1,1-dimethylbutyl)-6-(1 '-hydroxyethyl)-7-oxa-1-azabicyclo[3.2.0]hept-2-ene-2-carboxylic acid | 250 mg |
| | |
| Ceftazidime | 100 mg |
| | |
| Benzalkonium chloride | 0.1 mg |
| | |
| Sterile water (is added from a separate ampoule Using a syringe immediately before use) | 2 ml |
| | |

| Topical cream or ointment | |
|---|---|
| (4 : 1) co-lyophilizate of lactose monohydrate and (5R,6R,1'R)-3-(4-amino-1,1-dimethylbutyl)-6-(1'-hydroxyethyl)-7-oxa-1-azabicyclo[3.2.0]hept-2-ene-2-carboxylic acid | 250 mg |
| | |
| Ceftazidime | 100 mg |
| | |
| Polyethylene glycol 4000 V.S.P. | 800 mg |
| | |
| Polyethylene glycol 400 V.S.P. | 200 mg |

The active component in the above preparations can be mixed alone or together with other biologically active components, such as lincomycin, a penicillin, streptomycin, novobiocin, gentamycin, neomycin, colistin and kanamycin, or with other therapeutic agents such as probenicid.

It is understood that the specification and examples are illustrative but not limitative of the present invention and that other embodiments within the spirit ona scope of the invention will suggest themselves to those skilled in the art.

## Claims

1. A pharmaceutical composition comprising a co-lyophilizate of : a pharmaceutical carrier; and an active ingredient of formulae I and II or a pharmaceutically acceptable salt thereof, 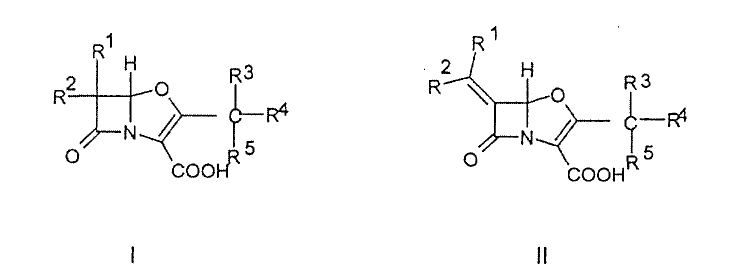 wherein R¹ and R², independently of one another, denote hydrogen, or pharmaceutically acceptable groups which have 1 to 10 carbon atoms and are bonded to the remaining part of the molecule via carbon-carbon single bonds and in which R³, R⁴ and R⁵, independently of one another, denote pharmaceutically acceptable groups which are bonded to the exocyclic, allylic carbon atom via carbon atoms.

2. Pharmaceutical composition according to claim 1, wherein R¹ and R² independently of one another, are selected from : hydrogen, or the pharmaceutically acceptable groups which are bonded to the remaining part of the molecule by C-C single bonds and which contain: substituted or unsubstituted alkyl, alkenyl, alkinyl, cycloalkyl, alkylcycloalky, alkylcycloalkenyl, cycloalkylalkyl, alkenylcycloalkyl, cycloalkenylalkyl, aryl, aralkyl, aralkenyl, aralkinyl, carboxyl or cyano, wherein the foregoing alkyl, alkenyl or alkinyl molecular parts contain 1 to 6 carbon atoms, and the cycloalkyl or cycloalkenyl molecule parts contain 3 to 6 carbon atoms and the aryl molecular parts contain 6 to 10 carbon atoms, heteroaryl, heteroaralkyl, heteroaralkenyl, heteroaralkinyl, alkylheteroaryl, heterocyclyl, heterocyclylalkyl, heterocyclyalkenyl, heterocyclylalkinyl, or alkylheterocyclyl, wherein the foregoing alkyl, alkenyl or alkinyl molecular parts contain 1 to 6 carbon atoms and the heteroaromatic or heterocyclic molecule part is monocyclic or bicyclic and contains 3 to 10 ring atoms, of which one or more are selected from the series comprising: oxygen, sulfur and nitrogen, and wherein the substituents of the above mentioned groups may be: protected or unprotected hydroxyl, hydroxyalkoxy, aminoalkoxy, amidinoalkoxy, alkoxy, acyloxy, aryloxy, heteroaryloxy, heterocyclyloxy, carbamoyl, carbamoyloxy, thiocarbamoyl, thiocarbamoyloxy, alkylcarbamoyloxy, alkylthiocarbamoyloxy, mercapto, alkylthio, hydroxyalkylthio, aminoalkylthio, amidinoalkylthio, acylthio, arylthio, alkylheteroarylthio, hydroxyalkylheteroarylthio, heterocyclylthio, carbamoylthio, alkylcarbamoylthio, thiocarbamoylthio or alkylthiocarbamoylthio, protected or unprotected amino or monoalkylamino, dialkylamino, oxo, protected or unprotected oximino or alkylamino, tetraalkylammonium, cycloalkylamino, arylamino, heteroarylamino, heterocyclylamino, acylamino, amidino, alkylamidino, guanidino, alkyguanidino, carbamoylamino, alkylcarbamoylamino, thiocarbamoylamino, alkylthiocarbamoylamino, nitro, chloro, bromo, fluoro, iodo, azido, cyano, alkylsulphinyl, alkylsulphonyl, sulphonamido, sulphamoyloxy, alkylsulphonyloxy, or protected or unprotected sulpho, sulphoxy or carboxyl, wherein the substituents, indpendently of one another, occur once or several times and their alkyl molecule part contains 1 to 6 carbon atoms and their aryl molecule part contains 6 to 10 carbon atoms, and wherein the heteroaromatic or heterocyclic molecule part is monocyclic or bicyclic and contains 3 to 10 ring atoms, of which one or more are selected from the series comprising: oxygen, sulphur and nitrogen, and **characterized in that** R³, R⁴ and R⁵, independently of one another, are selected from the above mentioned, pharmaceutically acceptable groups which are bonded to the remaining part of the molecule via carbon-carbon single bonds..

3. A pharmaceutical composition according to claim 1 or 2 wherein the pharmaceutical carrier is lactose, saccharose, glucose, corn starch or maize starch.

4. A pharmaceutical composition according to any of claims 1, 2 or 3 further comprising an additional active constituent.

5. A pharmaceutical composition according to claim 4 comprising a co-lyophilizate of the pharmaceutical carrier; the active ingredient and the additional active constituent.

6. A pharmaceutical composition according to any preceding claim in unit dose form.

7. Use of a composition according to any preceding claim in the manufacture of a medicament for the treatment of infection.

8. A process for preparation of a pharmaceutical composition according to any of claims 1 to 6 comprising the step of co-lyophilizating the pharmaceutical carrier and the active ingredient.

9. A method of treatment of an infection in a patient in need thereof which comprises administering to such a patient a pharmaceutical composition according to any of claims 1 to 6.

10. A method of improving the shelf life of a pharmaceutical composition which includes an active ingredient comprising the step of lyophilizing a pharmaceutical carrier and an active ingredient, in which the active ingredient comprises an ingredient of formulae I or II, or pharmaceutically acceptable salt thereof: 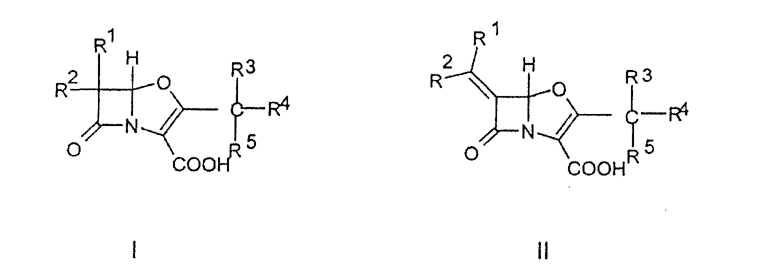 wherein R¹ and R², independently of one another, denote hydrogen, or pharmaceutically acceptable groups which have 1 to 10 carbon atoms and are bonded to the remaining part of the molecule via carbon-carbon single bonds and in which R³, R⁴ and R⁵, independently of one another, denote pharmaceutically acceptable groups which are bonded to the exocyclic, allylic carbon atom via carbon atoms.
